(19) Europäisches Patentamt
European Patent Office
Office européen des brevets

(11) Veröffentlichungsnummer : **0 161 455**
**B1**

(12) # EUROPÄISCHE PATENTSCHRIFT

(45) Veröffentlichungstag der Patentschrift : **15.06.88**

(21) Anmeldenummer : **85103953.7**

(22) Anmeldetag : **02.04.85**

(51) Int. Cl.⁴ : **C 07 D265/30**, C 07 D211/14, C 07 D295/02, C 07 C 87/28, A 01 N 33/04, A 01 N 33/10, A 01 N 43/40, A 01 N 43/84

(54) **Beta-naphthylalkylamine.**

(30) Priorität : **13.04.84 DE 3413897**

(43) Veröffentlichungstag der Anmeldung : **21.11.85 Patentblatt 85/47**

(45) Bekanntmachung des Hinweises auf die Patenterteilung : **15.06.88 Patentblatt 88/24**

(84) Benannte Vertragsstaaten : **AT BE CH DE FR GB IT LI NL SE**

(56) Entgegenhaltungen :
**EP-A- 0 007 479**
**FR-A- 2 371 436**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Weissmüller, Joachim, Dr.**
**Carl-Langhaus-Strasse 53**
**D-4019 Monheim (DE)**
Erfinder : **Brandes, Wilhelm, Dr.**
**Eichendorffstrasse 3**
**D-5653 Leichlingen (DE)**
Erfinder : **Hänssler, Gerd, Dr.**
**Heymannstrasse 40**
**D-5090 Leverkusen 1 (DE)**
Erfinder : **Reinecke, Paul, Dr.**
**Lessingstrasse 11**
**D-5090 Leverkusen 3 (DE)**

**EP 0 161 455 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue β-Naphthylalkylamine, mehrere Verfahren zu ihrer Herstellung und ihre Verwendung als Fungizide.

Es ist bereits bekannt, daß bestimmte Arylalkylaminoverbindungen, wie beispielsweise das 1-(4-t-Butylphenyl)-1-hydroxy-2-methyl-3-piperidin-1-yl-propan Hydrochlorid oder das 1-(4-t-Butylphenyl)-1-hydroxy-2-methyl-3-morpholin-4-yl-propan Hydrochlorid oder das 1-(4-t-Butylphenyl)-1-brom-2-methyl-3-(2,6-dimethylmorpholin-4-yl)-propan, fungizide Eigenschaften besitzen (vgl. z. B. DE-OS 3 019 496).

Die Wirkung dieser Verbindungen ist jedoch unter bestimmten Bedingungen, insbesondere bei niedrigen Aufwandmengen und Konzentrationen, nicht immer in allen Anwendungsbereichen völlig zufriedenstellend.

Ferner sind N-Phenylpropylsubstituierte cyclische Amine mit fungizider Wirksamkeit bekannt (vgl. EP-A-7479).

Weiterhin sind N-[4-tert.-Butylphenyl- bzw. 4-tert.-Butylhexyl]-propyl-substituierte Stickstoffheterocyclen bekannt, die eine fungizide Wirksamkeit haben (vgl. FR-A-2 371 436).

Es wurden neue β-Naphthylalkylamine der allgemeinen Formel (I)

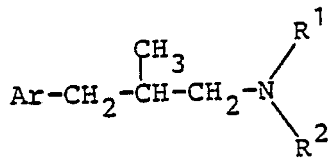

(I)

in welcher

Ar für gegebenenfalls ein- bis dreifach, gleich oder verschieden substituiertes β-Naphthyl steht, wobei als Substituenten in Frage kommen : Halogen, Hydroxy sowie jeweils geradkettiges oder verzweigtes Alkyl oder Alkoxy mit jeweils 1 bis 6 Kohlenstoffatomen und

$R^1$ und $R^2$, welche gleich oder verschieden sein können, für jeweils geradkettiges oder verzweigtes Alkyl oder Alkenyl mit jeweils bis zu 6 Kohlenstoffatomen stehen oder

$R^1$ und $R^2$ gemeinsam mit dem Stickstoff, an welches sie gebunden sind, für einen gegebenenfalls ein- bis dreifach, gleich oder verschieden substituierten, gesättigten fünf- bis siebengliedrigen Heterocyclus stehen, der 1 oder 2 weitere Heteroatome, insbesondere Stickstoff oder Sauerstoff enthalten kann, wobei als Substituenten in Frage kommen : jeweils geradkettiges oder verzweigtes Alkyl oder Hydroxyalkyl mit jeweils 1 bis 4 Kohlenstoffatomen,

sowie deren pflanzenverträgliche Säureadditionssalze gefunden.

Weiterhin wurde gefunden, daß man die neuen β-Naphthylalkylamine der allgemeinen Formel (I)

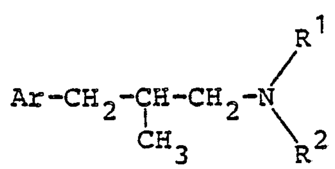

(I)

in welcher

Ar für gegebenenfalls ein- bis dreifach, gleich oder verschieden substituiertes β-Naphthyl steht, wobei als Substituenten in Frage kommen : Halogen, Hydroxy sowie jeweils geradkettiges oder verzweigtes Alkyl oder Alkoxy mit jeweils 1 bis 6 Kohlenstoffatomen und

$R^1$ und $R^2$, welche gleich oder verschieden sein können, für jeweils geradkettiges oder verzweigtes Alkyl oder Alkenyl mit jeweils bis zu 6 Kohlenstoffatomen stehen oder

$R^1$ und $R^2$ gemeinsam mit dem Stickstoff, an welches sie gebunden sind, für einen gegebenenfalls ein- bis dreifach, gleich oder verschieden substituierten, gesättigten fünf- bis siebengliedrigen Heterocyclus stehen, der 1 oder 2 weitere Heteroatome, insbesondere Stickstoff oder Sauerstoff enthalten kann, wobei als Substituenten in Frage kommen : jeweils geradkettiges oder verzweigtes Alkyl oder Hydroxyalkyl mit jeweils 1 bis 4 Kohlenstoffatomen,

sowie deren pflanzenverträgliche Säureadditionssalze erhält, wenn man

(a) β-Naphthylalkyl-Verbindungen der Formel (II)

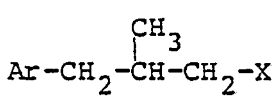

(II)

in welcher

Ar die oben angegebene Bedeutung hat und
X für eine elektronenanziehende Austrittsgruppe steht,
mit Aminen der Formel (III)

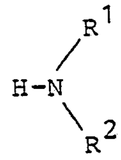

(III)

in welcher $R^1$ und $R^2$ die oben angegebene Bedeutung haben,
gegebenenfalls in Gegenwart eines Verdünnungsmittels und gegebenenfalls in Gegenwart eines Säurebindemittels umsetzt, oder wenn man

(b) ungesättigte Aldehyde der Formel (IV)

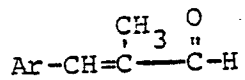

(IV)

in welcher Ar die oben angegebene Bedeutung hat, mit Aminen der Formel (III)

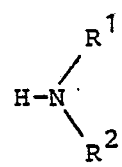

(III)

in welcher $R^1$ und $R^2$ die oben angegebene Bedeutung haben,
in Gegenwart eines Reduktionsmittels und gegebenenfalls in Gegenwart eines Katalysators sowie gegebenenfalls in Gegenwart eines Verdünnungsmittels umsetzt, und gegebenenfalls anschließend eine Säure addiert.

Außerdem ist es möglich, die erfindungsgemäßen β-Naphthylalkylamine der Formel (I) nach allgemein üblichen Methoden am Stickstoff zu den entsprechenden tetrasubstituierten Ammoniumsalzen zu quaternieren.

Schließlich wurde gefunden, daß die neuen β-Naphthylalkylamine der Formel (I) fungizide Eigenschaften besitzen.

Dabei zeigen die erfindungemäßen β-Naphthylalkylamine der Formel (I) überraschenderweise eine erheblich bessere fungizide Wirksamkeit, als die aus dem Stand der Technik bekannten Arylalkylaminoverbindungen, wie beispielsweise das 1-(4-t-Butylphenyl)-1-hydroxy-2-methyl-3-piperidin-1-yl-propan Hydrochlorid oder das 1-(4-t-Butylphenyl)-1-hydroxy-2-methyl-3-morpholin-4-yl-propan Hydrochlorid oder das 1-(4-t-Butylphenyl)-1-hydroxy-2-methyl-3-(2,6-dimethylmorpholin-4-yl)-propan oder das 1-(4-t-Butylphenyl)-1-brom-2-methyl-3-(2,6-dimethyl-morpholin-4-yl)-propan, welches chemisch und wirkungsmäßig naheliegende Verbindungen sind.

Die erfindungsgemäßen β-Naphthylalkylamine sind durch die Formel (I) allgemein definiert. Bevorzugt sind Verbindungen der Formel (I), bei welchen

Ar für gegebenenfalls einfach oder zweifach, gleich oder verschieden substituiertes β-Naphthyl steht, wobei als Substituenten in Frage kommen : Fluor, Chlor, Brom, Hydroxy, Methyl, Ethyl, n- oder i-Propyl, n-, i-, s- oder t-Butyl, Methoxy, Ethoxy, n- oder i-Propoxy und

$R^1$ und $R^2$, welche gleich oder verschieden sein können, für Methyl, Ethyl, n- oder i-Propyl, Allyl, Butenyl, Dimethylallyl sowie n- oder i-Pentenyl stehen oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

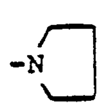 , 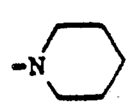 , 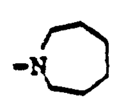 oder 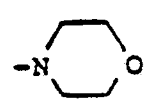

stehen, wobei als substituenten in Frage kommen : Methyl, Ethyl oder Hydroxymethyl.

Im einzelnen seien außer den bei den Herstellungsbeispielen genannten Verbindungen die folgenden β-Naphthylalkyl-amine der Formel (I) genannt :

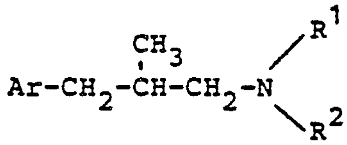 (I)

| Ar | $R^1$ | $R^2$ bzw. 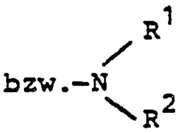 |
|---|---|---|
| 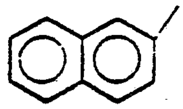 | $CH_3$ | $-CH_2-CH=CH_2$ |
| 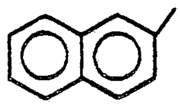 | — | — 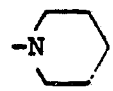 |
| 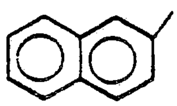 | — | — 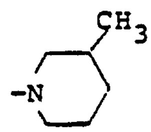 |
| 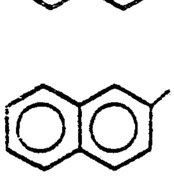 | — | — 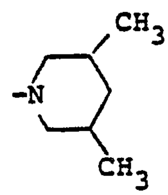 |
| 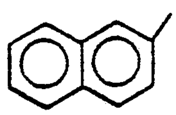 | $CH_3$ | 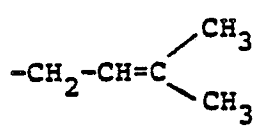 |
| 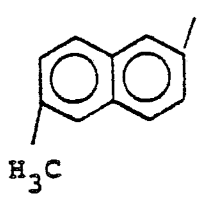 | — | — 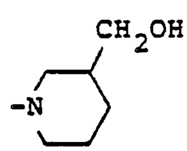 |
| 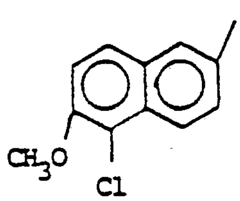 | — | — 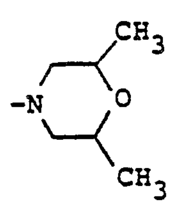 |

(Fortsetzung)

| Ar | $R^1$ | $R^2$ | bzw. 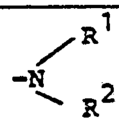 |
|---|---|---|---|
| 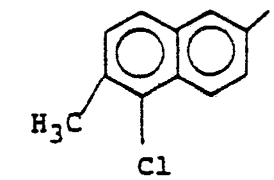 | — | — | 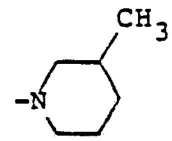 |
| 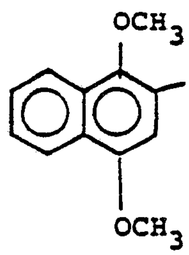 | $CH_3$ | 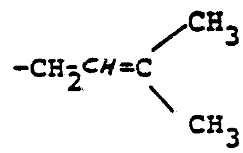 | |
| 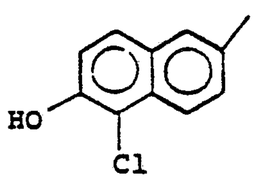 | — | — | 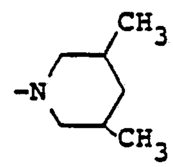 |
| 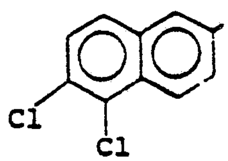 | — | — | 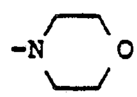 |
| 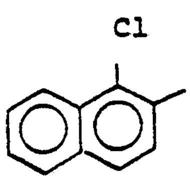 | $CH_3$ | $-CH_2-CH=CH_2$ | |
| 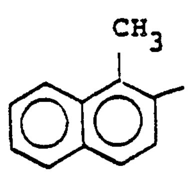 | — | — | 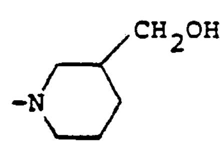 |

(Fortsetzung)

| Ar | $R^1$ | $R^2$ | bzw. 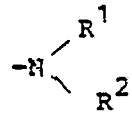 |
|---|---|---|---|
| 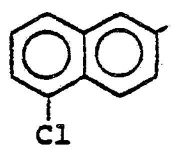 | – | – | 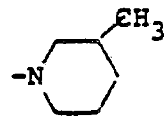 |
| 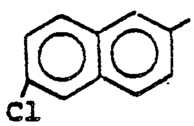 | – | – | 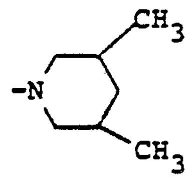 |

Verwendet man als Ausgangsstoffe beispielsweise 1-Methansulfonyloxy-2-methyl-3-β-naphthyl-propan und 2,6-Dimethylmorpholin, so läßt sich der Reaktionsablauf des erfindungsgemäßen Verfahrens (a) durch das folgende Formelschema darstellen :

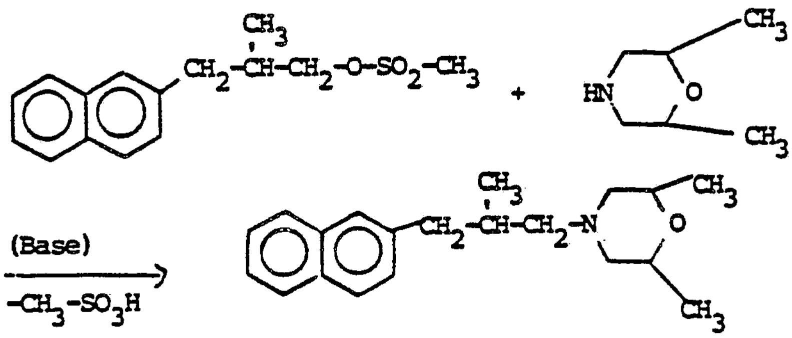


Verwendet man als Ausgangsstoffe beispielsweise 2-Methyl-3-β-naphthyl-acrolein und 3,5-Dimethylpiperidin sowie Wasserstoff als Reduktionsmittel, so läßt sich der Reaktionsablauf des erfindungsgemäßen Verfahrens (b) durch das folgende Formelschema darstellen :

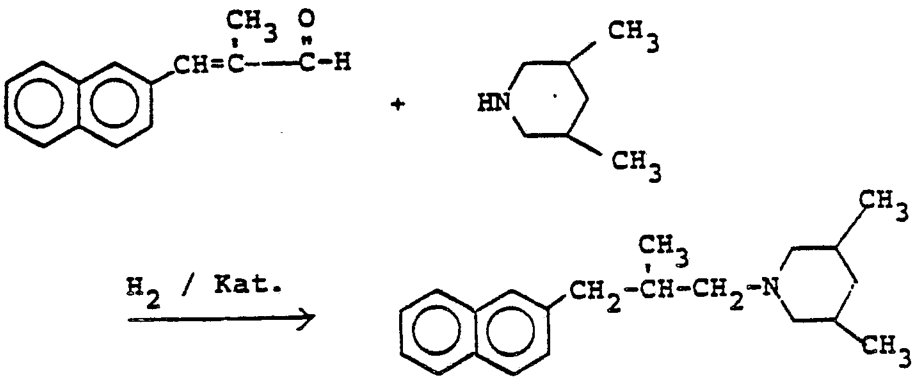


Die zur Durchführung des erfindungsgemäßen Verfahrens (a) als Ausgangsstoffe benötigten β-Naphthylalkylverbindungen sind durch die Formel (II) allgemein definiert. In dieser Formel (II) steht Ar vorzugsweise für diejenigen gegebenenfalls substituierten β-Naphthylreste, die schon bei der Beschreibung der erfindungsgemäßen Stoffe der Formel (I) als bevorzugt für diesen Rest genannt werden. X steht vorzugsweise für Halogen, insbesondere Chlor, Brom oder Jod, oder für jeweils gegebenenfalls substituiertes Alkyl- oder Arylsulfonyloxy, insbesondere für Methansulfonyloxy, Trifluormethansulfonyloxy oder p-Toluol-sulfonyloxy.

Die β-Naphtylalkylverbindungen der Formel (II) sind noch nicht bekannt.

Man erhält sie, wenn man 2-Methyl-3-β-naphthyl-acryl-ester der Formel (V)

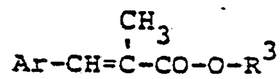 (V)

in welcher

Ar die oben angegebene Bedeutung hat und

$R^3$ für Alkyl, insbesondere für Methyl oder Ethyl, steht,

zunächst in einer 1. Stufe mit einem Reduktionsmittel, wie beispielsweise Lithiumaluminiumhydrid, gegebenenfalls in Gegenwart eines Verdünnungsmittels, wie beispielsweise Diethylether, bei Temperaturen zwischen — 20 °C und + 60 °C reduziert, und die so erhaltenen Alkohole der Formel (VI),

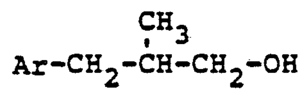 (VI)

in welcher Ar die oben angegebene Bedeutung hat, nach allgemein üblichen Verfahren derivatisiert, so zum Beispiel entweder mit Sulfonsäurehalogeniden der Formel (VII)

$$R^4—SO_2—Hal \qquad (VII)$$

in welcher

$R^4$ für jeweils gegebenenfalls substituiertes Alkyl oder Aryl, insbesondere für Methyl, Trifluormethyl oder p-Tolyl, steht, und

Hal für Halogen, insbesondere Chlor oder Brom, steht,

gegebenenfalls in Gegenwart eines Verdünnungsmittels, wie beispielsweise Dichlormethan, und gegebenenfalls in Gegenwart eines Säurebindemittels, wie beispielsweise Triethylamin, bei Temperaturen zwischen — 20 °C und + 120 °C sulfoniert oder mit Halogenierungsmitteln wie Thionylchlorid, Phosphorpentachlorid, Phosphortribromid, Bromwasserstoffsäure oder Jodwasserstoffsäure gegebenenfalls in Gegenwart eines Verdünnungsmittels, wie beispielsweise Tetrachlormethan, und gegebenenfalls in Gegenwart eines Katalysators, wie beispielsweise Pyridin, bei Temperaturen zwischen + 20 °C und + 180 °C halogeniert.

Die 2-Methyl-3-β-naphthylacrylester der Formel (V) sind bekannt (vgl. z. B. Indian J. Chem. Section B, 22B (4), 352-354 [1983] oder J. org. Chemistry 33, 4351-4362 [1968]) oder lassen sich nach prinzipiell bekannten Verfahren in einfacher analoger Weise herstellen (vgl. z. B. J. Chem. Soc. 1961, 3160). Die Sulfonsäurehalogenide der Formel (VII) sind allgemein bekannte Verbindungen der organischen Chemie.

Die zur Durchführung der erfindungsgemäßen Verfahrens (b) als Ausgangsstoffe benötigten ungesätttigten Aldehyde sind durch die Formel (IV) allgemein definiert. In dieser Formel (IV) steht Ar vorzugsweise für diejenigen gegebenenfalls substituierten β-Naphthylreste, die schon bei der Beschreibung der erfindungsgemäßen Stoffe der Formel (I) als bevorzugt für diesen Rest genannt werden.

Die ungesättigten Aldehyde der Formel (IV) sind noch nicht bekannt. Man erhält sie jedoch nach prinzipiell bekannten Verfahren, wenn man β-Naphthaldehyde der Formel (VIII)

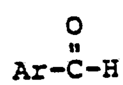 (VIII)

in welcher Ar die oben angegebene Bedeutung hat,

mit Propionaldehyd der Formel (IX)

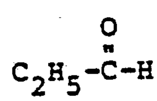 (IX)

gegebenenfalls in Gegenwart eines Verdünnungsmittels, wie beispielsweise Methanol, und gegebenenfalls in Gegenwart eines Katalysators, wie beispielsweise Natriumhydroxid, bei Temperaturen zwischen — 20 °C und + 120 °C umsetzt und aus den so erhältlichen Additionsprodukten der Formel (X)

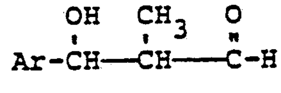 (X)

in welcher Ar die oben angegebene Bedeutung hat,

ebenfalls in allgemein üblicher Art und Weise mit einem sauren Katalysator, wie beispielsweise Essigsäure, gegebenenfalls in Gegenwart eines Verdünnungsmittels, wie beispielsweise Methanol, bei Temperaturen zwischen + 20 °C und 180 °C Wasser abspaltet.

Die β-Naphthaldehyde der Formel (VIII) und der Propionaldehyd der Formel (IX) sind allgemein bekannte Verbindungen der organischen Chemie.

Die zur Durchführung der erfindungsgemäßen Verfahren (a) und (b) weiterhin als Ausgangsstoffe benötigten Amine sind durch die Formel (III) allgemein definiert. In dieser Formel (III) stehen $R^1$ und $R^2$ vorzugsweise für diejenigen Reste, die schon bei der Beschreibung der erfindungsgemäßen Stoffe der Formel (I) als bevorfür diese Substituenten genannt wurden.

Die Amine der Formel (III) sind ebenfalls allgemein bekannte Verbindungen der organischen Chemie.

Als Verdünnungsmittel zur Durchführung des erfindungsgemäßen Verfahrens (a) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid. Es kann jedoch auch ohne Verdünnungsmittel gearbeitet werden.

Das erfindungsgemäße Verfahren (a) kann gegebenenfalls in Gegenwart eines Säurebindemittels durchgeführt werden.

Als solche kommen alle üblichen anorganischen oder organischen Basen in Frage. Hierzu gehören beispielsweise Alkalimetallhydroxide, wie Natriumhydroxid oder Kaliumhydroxid ; Alkalimetallcarbonate, wie Natriumcarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Ein entsprechender Überschuß an Amin der Formel (III), welches als Reaktionspartner verwendet wird, kann gleichzeitig als Säurebindemittel und, falls das Amin in flüssiger Form vorliegt, auch als Verdünnungsmittel dienen.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und 250 °C, vorzugsweise bei Temperaturen zwischen 30 °C und 180 °C.

Zur Durchführung des erfindungsgemäßen Verfahrens (a) setzt man pro Mol β-Naphthylalkyl-Verbindung der Formel (II) im allgemeinen 1,0 bis 10,0 Mol, vorzugsweise 1,0 bis 5,0 Mol, an Amin der Formel (III) und gegebenenfalls 1,0 bis 10,0 Mol, vorzugsweise 1,0 bis 5,0 Mol, an Säurebindemittel ein. Zur Aufarbeitung wird das Reaktionsprodukt von wasserlöslichen Verunreinigungen durch Verteilung in einem wäßrig-organischen Zweiphasensystem abgetrennt, wie allgemein üblich isoliert und anschließend gegebenenfalls durch Säulenchromatographie gereinigt.

Als Verdünnungsmittel zur Durchführung des erfindungsgemäßen Verfahren (b) kommen ebenfalls inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole, wie Methanol, Ethanol oder Isopropanol. Es kann jedoch auch ohne Verdünnungsmittel gearbeitet werden.

Als Reduktionsmittel kommen alle üblichen Carbonylgruppen reduzierenden Verbindungen in Frage. Vorzugsweise verwendet man molekularen Wasserstoff oder Ameisensäure.

Als Katalysatoren kommen ebenfalls alle üblichen Hydrierkatalysatoren in Frage. Vorzugsweise verwendet man Edelmetall-, Edelmetalloxid- oder Edelmetallhydroxid-katalysatoren oder sogenannte Raney-Katalysatoren, insbesondere Platin, Platinoxid und Raney-Nickel.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und + 250 °C, vorzugsweise bei Temperaturen zwischen + 20 °C und + 200 °C.

Das erfindungsgemäße Verfahren (b) kann bei Normaldruck oder auch bei erhöhtem Druck durchgeführt werden. Im allgemeinen arbeitet man zwischen 1 atm und 300 atm, vorzugsweise zwischen 1 atm und 200 atm.

Zur Durchführung des erfindungsgemäßen Verfahrens (b) setzt man pro Mol an ungesättigtem Aldehyd der Formel (IV) im allgemeinen 1,0 bis 5,0 Mol, vorzugsweise 1,0 bis 2,0 Mol, an Amin der Formel (III) und 1,0 bis 10 Mol, vorzugsweise 1,0 bis 5,0 Mol, an Reduktionsmittel sowie gegebenenfalls 0,01 bis 0,1 Mol an Katalysator ein. Die Aufarbeitung und Isolierung der Verbindungen der Formel (I) erfolgt in allgemein üblicher Art und Weise.

Die erfindungsgemäßen Verbindungen der Formel (I) können gegebenenfalls anschließend in Säureadditionssalze überführt werden.

Zur Herstellung von physiologisch verträglichen Säureadditionssalzen der Verbindungen der Formel (I) kommen vorzugsweise folgende Säuren in Frage : Halogenwasserstoffsäuren, wie z. B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditionssalze der Verbindungen der Formel (I) können in einfacher Weise nach üblichen Salzbildungsmethoden, wie z. B. durch Lösen einer Verbindung der Formel (I) in einem geeigneten inerten Lösungsmittel und Hinzufügen der Säure, wie z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So werden z. B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe können mit besonders gutem Erfolg zur Bekämpfung von Getreidekrankheiten, wie beispielsweise gegen den Erreger der Braunfleckigkeit des Weizen (Leptosphaeria nodorum), gegen den Erreger der Streifenkrankheit der Gerste (Drecksiera graminea), gegen den Erreger der Netzfleckenkrankheit der Gerste (Pyrenophora teres), sowie gegen den Erreger des echten Getreidemehltaus (Erysiphe graminis), zur Bekämpfung von Reiskrankheiten, wie beispielsweise gegen den Erreger der Reisfleckenkrankheit (Pyricularia oryzae) oder zur Bekämpfung von Gemüsekrankheiten, wie beispielsweise gegen den Erreger des echtern Gurkenmehltaus (Sphaerotheca fuliginea) eingesetzt werden. Dabei zeigen die erfindungsgemäßen Wirkstoffe neben einer hervorragenden protektiven Wirksamkeit auch äußerst gute systemische Eigenschaften.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Tolyol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten

Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Herstellungsbeispiele

### Beispiel 1

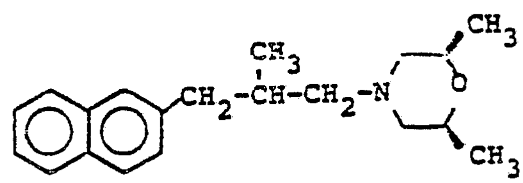

10,8 g (0,04 Mol) 1-Methansulfonyl-2-methyl-3-β-naphthylpropan und 9 g (0,078 Mol) cis-2,6-Dimethylmorpholin werden bei einer Badtemperatur von 140 °C 15 Stunden gerührt. Die erhaltene Reaktionsmischung wird mit Wasser versetzt und mehrfach mit Ether extrahiert. Die vereinigten organischen Phasen werden über Natriumsulfat getrocknet und im Vakuum vom Lösungsmittel befreit ; der ölige Rückstand wird säulenchromatographisch (Kieselgel 60/Petrolether-Ether 2 : 1) gereinigt. Man erhält 6,2 g (52 % der Theorie) an cis-1-(2,6-Dimethylmorpholin-4-yl)-2-methyl-3-β-naphthyl-propan vom Brechungsindex $n_D^{20}$ : 1.5527.

Herstellung der Ausgangsverbindung :

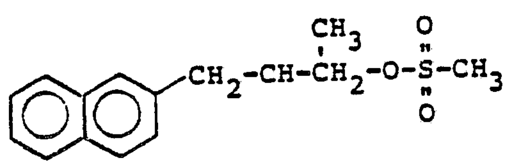

Zu 14 g (0,074 Mol) 2-Methyl-3-β-naphthylpropanol (roh) in 80 ml absolutem Pyridin gibt man bei 0 °C tropfenweise unter Rühren 11 g (0,1 Mol) Methansulfonsäurechlorid, rührt nach beendeter Zugabe weitere 16 Stunden bei Raumtemperatur, entfernt überschüssiges Pyridin durch Destillation im Vakuum, nimmt den Rückstand in Wasser auf, extrahiert mehrfach mit Dichlormethan, trocknet über Natriumsulfat und entfernt das Lösungsmittel im Vakuum. Man erhält 13,6 g (66 % der Theorie) an 1-Methansulfonyloxy-2-methyl-3-β-naphthyl-propan als Öl. (IR : $\gamma$ = 1 345, 1 180 $cm^{-1}$).

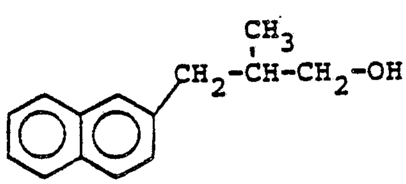

12 g (0,05 Mol) 2-Methyl-3-β-naphthylacrylsäureethylester tropft man unter Eiskühlung in einer trockenen Stickstoffatmosphäre zu einer Suspension von 1,9 g (0,05 Mol) Lithiumaluminiumhydrid in 150 ml absolutem Ether. Nach beendeter Zugabe erwärmt man 8 Stunden lang auf Rückflußtemperatur und tropft dann nach Erkalten der Reaktionsmischung langsam unter Kühlung 15 ml 5-prozentige Schwefelsäure zu, saugt den ausgefallenen Feststoff ab, trocknet das Filtrat über Natriumsulfat, entfernt das Lösungsmittel im Vakuum und kristallisiert den Rückstand aus Ether/Petrolether um. Man erhält 7,1 g 2-Methyl-3-β-naphthyl-propanol vom Schmelzpunkt 71-74 °C, das laut Gaschromatogram mit 2-Methyl-1-β-

naphthyl-propen-3-ol verunreinigt ist und ohne weitere Reinigung in die nächste Reaktionsstufe eingesetzt wird.

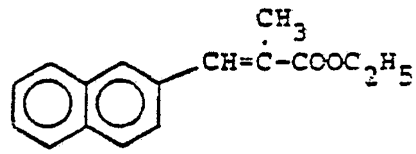

Zu einer Suspension von 5,5 g (0,2 (Mol)) 80 %igem Natriumhydrid in 300 ml absolutem Xylol gibt man bei 70 °C 40 g (0,2 Mol) Ethyl-α-ethoxalylpropionat. Nach beendeter Wasserstoffentwicklung tropft man 31,2 g (0,2 Mol) β-Naphthaldehyd in Xylol gelöst zu und erwärmt nach beendeter Zugabe für 90 Minuten zum Sieden.

Die erkaltete Reaktionsmischung wird mit 150 ml Wasser versetzt, die organische Phase abgetrennt, mit 7-%iger Natriumcarbonatlösung gewaschen, über Natriumsulfat getrocknet und eingeengt. Der Rückstand wird im Vakuum destilliert. Man erhält 21,7 g (45,2 % der Theorie) an 2-Methyl-3-β-naphthyl-acrylsäureethylester vom Siedepunkt 110 °C/0,13 mbar.

Beispiel 2

Analog Beispiel 1 wird die folgende Verbindung hergestellt :

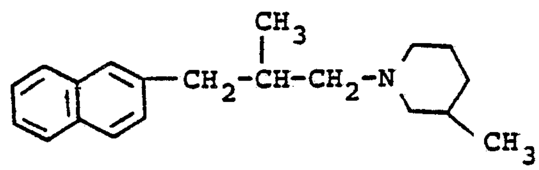

mit einem Brechungsindex $n_D^{20}$ : 1.5547.

Anwendungsbeispiele

In den folgenden Anwendungsbeispielen werden die nachstehend aufgeführten Verbindungen als Vergleichssubstanzen eingesetzt :

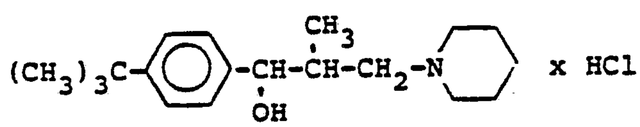
(A)

1-(4-t-Butylphenyl)-1-hydroxy-2-methyl-3-piperidin-1-yl-propan Hydrochlorid

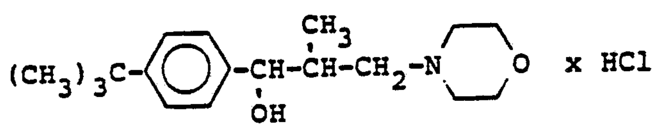
(B)

1-(4-t-Butylphenyl)-1-hydroxy-2-methyl-3-morpholin-4-yl-propan Hydrochlorid

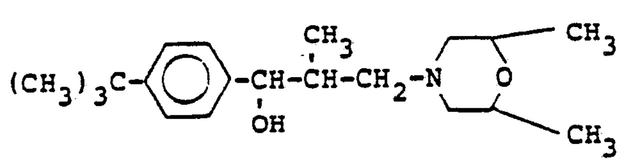
(C)

1-(4-t-Butylphenyl)-1-hydroxy-2-methyl-3-(2,5-dimethyl-morpholin-4-yl)-propan

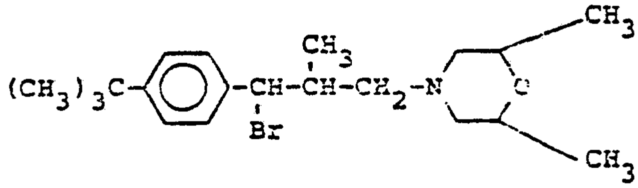
(D)

1-Brom-1-(4-t-butylphenyl)-2-methyl-3-(2,6-dimethyl-morpholin-4-yl)-propan (alle bekannt aus DE-OS 30 19 496)

Beispiel A

Leptosphaeria nodorum-Test (Weizen)/protektiv

Lösungsmittel : 100 Gew.-Teile Dimethylformamid
Emulgator : 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufecht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel 1.

Beispiel B

Pyrenophora teres-Test (Gerste)/protektiv

Lösungsmittel : 100 Gew.-Teile Dimethylformamid
Emulgator : 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z. B. die Verbindung gemäß Herstellungsbeispiel 1.

Beispiel C

Drechslera graminea-Test (Gerste)/Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 × 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigt in diesem Test z. B. die Verbindung gemäß Herstellungsbeispiel 1.

Beispiel D

Sphaerotheca-Test (Gurke)/protektiv

Lösungsmittel : 4,7 Gewichtsteile Aceton
Emulgator : 0,3 Gewichtsteile Alkylaryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigt in diesem Test z. B. die Verbindung gemäß Herstellungsbeispiel 1.

Beispiel E

Pyricularia-Test (Reis)/systemisch

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Tecknik zeigt in diesem Test z. B. die Verbindung gemäß Herstellungsbeispiel 1.

**Patentansprüche**

1. β-Naphthylalkylamine der Formel (I)

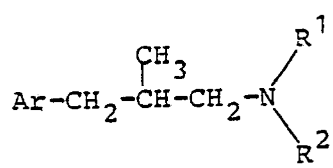

(I)

in welcher

Ar für gegebenenfalls ein- bis dreifach, gleich oder verschieden durch Halogen, Alkyl oder Alkoxy mit jeweils 1 bis 6 Kohlenstoffatomen substituiertes β-Naphthyl steht,

$R^1$ und $R^2$, welche gleich oder verschieden sein können für jeweils geradkettiges oder verzweigtes Alkyl oder Alkenyl mit jeweils bis zu 6 Kohlenstoffatomen stehen oder

$R^1$ und $R^2$ gemeinsam mit dem Stickstoffatom, an welches sie gebunden sind, für einen gegebenenfalls ein- bis dreifach, gleich oder verschieden durch jeweils geradkettiges oder verzweigtes Alkyl oder Hydroxyalkyl mit jeweils 1 bis 4 Kohlenstoffatomen substituierten gesättigten fünf- bis siebengliedrigen Heterocyclus stehen, der weitere 1 oder 2 Stickstoff- oder Sauerstoffatome enthalten kann.

2. β-Naphthylalkylamine gemäß Anspruch 1, wobei in der Formel (I)

Ar für gegebenenfalls einfach oder zweifach, gleich oder verschieden durch Fluor, Chlor, Brom, Hydroxy, Methyl, Ethyl, n- oder i-Propyl, n-, i-, s- oder t.-Butyl, Methoxy, Ethoxy, n- oder i-Propoxy substituiertes β-Naphthyl steht,

$R^1$ und $R^2$, welche gleich oder verschieden sein können, für Methyl, Ethyl, n- oder i-Propyl, Allyl, Dimethylallyl, Butenyl, n- oder i-Pentenyl stehen oder

$R^1$ und $R^2$ gemeinsam mit dem Stickstoffatom, an welches sie gebunden sind, für einen gegebenenfalls ein- bis dreifach, gleich oder verschieden durch Methyl, Ethyl oder Hydroxymethyl substituierten Heterocyclus der Formel

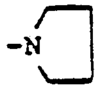 , 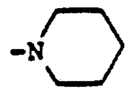 , 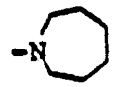 oder 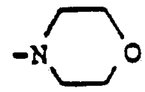

stehen.

3. Verfahren zur Herstellung von β-Naphthylalkylaminen der Formel (I)

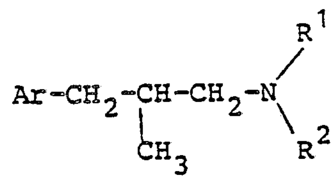 (I)

in welcher

Ar für gegebenenfalls ein- bis dreifach, gleich oder verschieden durch Halogen, Alkyl oder Alkoxy mit jeweils 1 bis 6 Kohlenstoffatomen substituiertes β-Naphthyl steht,

$R^1$ und $R^2$, welche gleich oder verschieden sein können für jeweils geradkettiges oder verzweigtes Alkyl oder Alkenyl mit jeweils bis zu 6 Kohlenstoffatomen stehen oder

$R^1$ und $R^2$ gemeinsam mit dem Stickstoffatom, an welches sie gebunden sind, für einen gegebenenfalls ein- bis dreifach, gleich oder verschieden durch jeweils geradkettiges oder verzweigtes Alkyl oder Hydroxyalkyl mit jeweils 1 bis 4 Kohlenstoffatomen substituierten gesättigten fünf- bis siebengliedrigen Heterocyclus stehen, der weitere 1 oder 2 Stickstoff- oder Sauerstoffatome enthalten kann,

sowie deren pflanzenverträgliche Säureadditionssalze, dadurch gekennzeichnet, daß man

(a) β-Naphthylalkyl-Verbindungen der Formel (II)

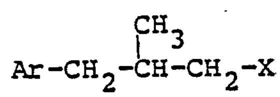 (II)

in welcher

Ar die oben angegebene Bedeutung hat und

X für eine elektronenanziehende Austrittsgruppe steht,

mit Aminen der Formel (III)

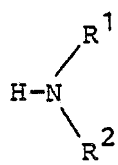 (III)

in welcher $R^1$ und $R^2$ die oben angegebene Bedeutung haben,
gegebenenfalls in Gegenwart eines Verdünnungsmittels und gegebenenfalls in Gegenwart eines Säurebindemittels umsetzt, oder daß man

(b) ungesättigte Aldehyde der Formel (IV)

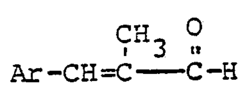 (IV)

in welcher Ar die oben angegebene Bedeutung hat,
mit Aminen der Formel (III)

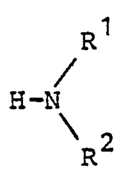 (III)

in welcher $R^1$ und $R^2$ die oben angegebene Bedeutung haben,
in Gegenwart eines Reduktionsmittels und gegebenenfalls in Gegenwart eines Katalysators sowie gegebenenfalls in Gegenwart eines Verdünnungsmittels umsetzt, und gegebenenfalls anschließend eine Säure addiert.

4. Fungizide, gekennzeichnet durch einen Gehalt an mindestens einem β-Naphthylalkylamin der Formel (I) gemäß den Ansprüchen 1 und 3.

5. Verwendung von β-Naphthylalkylaminen der Formel (I) gemäß den Ansprüchen 1 und 3 zur Bekämpfung von Pilzen, ausgenommen die vom Gesetz her nicht schutzfähigen Verwendungen.

6. Verfahren zur Bekämpfung von Pilzen, dadurch gekennzeichnet, daß man β-Naphthylalkylamine der Formel (I) gemäß den Ansprüchen 1 und 3 auf Pilze und/oder ihren Lebensraum einwirken läßt, ausgenommen die vom Gesetz her nicht schutzfähigen Verwendungen.

7. Verfahren zur Herstellung von fungiziden Mitteln, dadurch gekennzeichnet, daß man β-Naphthylalkylamine der Formel (I) gemäß den Ansprüchen 1 und 3 mit Streckmitteln und/oder oberflächenaktiven Mitteln vermischt.

**Claims**

1. β-Naphthylalkylamines of the formula (I)

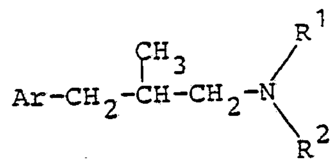 (I)

in which

Ar represents β-naphthyl which is optionally mono-, di- or trisubstituted by identical or different substituents from the group comprising halogen, alkyl and alkoxy with in each case 1 to 6 carbon atoms, and

$R^1$ and $R^2$, which can be identical or different, represent in each case straight-chain or branched alkyl or alkenyl with in each case up to 6 carbon atoms, or

$R^1$ and $R^2$, together with the nitrogen atom to which they are bonded, represent a saturated five-membered to seven-membered heterocyclic radical which is optionally mono-, di- or trisubstituted by identical or different substituents from the group comprising in each case straight-chain or branched alkyl and hydroxyalkyl with in each case 1 to 4 carbon atoms, and can contain a further 1 or 2 nitrogen or oxygen atoms.

2. β-Naphthylalkylamines according to Claim 1, wherein, in the formula (I),

Ar represents β-naphthyl which is optionally monosubstituted or disubstituted by identical or different substituents from the group comprising fluorine, chlorine, bromine, hydroxyl, methyl, ethyl, n- or i-propyl, n-, i-, s- or t.-butyl, methoxy, ethoxy and n- or i-propoxy, and

$R^1$ and $R^2$, which can be identical or different, represent methyl, ethyl, n- or i-propyl, allyl, dimethylallyl, butenyl or n- or i-pentenyl, or

$R^1$ and $R^2$, together with the nitrogen atom to which they are bonded, represent a heterocyclic radical of the formula

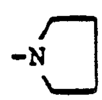 , 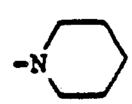 , 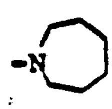 or 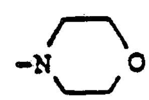

which is optionally mono-, di- or trisubstituted by identical or different substituents from the group comprising methyl, ethyl and hydroxymethyl.

3. Process for the preparation of β-naphthylalkylamines of the formula (I)

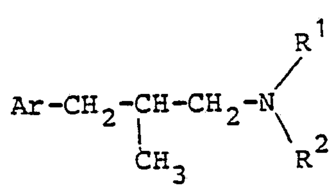 (I)

in which

Ar represents β-naphthyl which is optionally mono-, di- or trisubstituted by identical or different substituents from the group comprising halogen, alkyl and alkoxy with in each case 1 to 6 carbon atoms, and

$R^1$ and $R^2$, which can be identical or different, represent in each case straight-chain or branched alkyl or alkenyl with in each case up to 6 carbon atoms, or

$R^1$ and $R^2$, together with the nitrogen atom to which they are bonded, represent a saturated five-

membered to seven-membered heterocyclic radical which is optionally mono-, di- or trisubstituted by identical or different substituents from the group comprising in each case straight-chain or branched alkyl and hydroxyalkyl with in each case 1 to 4 carbon atoms, and can contain a further 1 or 2 nitrogen or oxygen atoms,
and acid addition salts thereof which are tolerated by plants, characterized in that

(a) β-naphthylalkyl compounds of the formula (II)

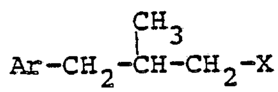

(II)

in which

Ar has the abovementioned meaning and

X represents an electron-withdrawing leaving group,

are reacted with amines of the formula (III)

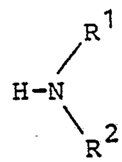

(III)

in which $R^1$ and $R^2$ have the abovementioned meaning,
if appropriate in the presence of a diluent and if appropriate in the presence of an acid-binding agent, or in that

(b) unsaturated aldehydes of the formula (IV)

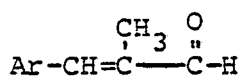

(IV)

in which Ar has the abovementioned meaning, are reacted with amines of the formula (III)

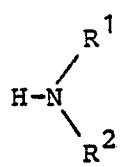

(III)

in which $R^1$ and $R^2$ have the abovementioned meaning, in the presence of a reducing agent and, if appropriate, in the presence of a catalyst, and, if appropriate, in the presence of a diluent, and, if appropriate, an acid is then added on.

4. Fungicides, characterized in that they contain at least one β-naphthylalkylamine of the formula (I) according to Claims 1 and 3.

5. Use of β-naphthylalkylamines of the formula (1) according to Claims 1 and 3 for combating fungi, except for the uses which cannot be protected by law.

6. Method of combating fungi, characterized in that β-naphthylalkylamines of the formula (I) according to Claims 1 and 3 are allowed to act on fungi and/or their environment, except for the uses which cannot be protected by law.

7. Process for the preparation of fungicidal agents, characterized in that β-naphthylalkylamines of the formula (I) according to Claims 1 and 3 are mixed with extenders and/or surface-active agents.

**Revendications**

1. β-naphtylalkylamines de formule (I)

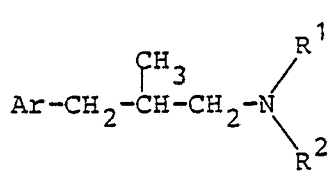

(I)

dans laquelle

Ar représente un reste β-naphtyle portant éventuellement un à trois substituants, identiques ou différents, halogéno, alkyle ou alkoxy ayant chacun 1 à 6 atomes de carbone,

$R^1$ et $R^2$, qui peuvent être identiques ou différents, représentent un groupe alkyle ou alcényle ayant chacun une chaîne droite ou ramifiée, avec jusqu'à 6 atomes de carbone, ou bien

$R^1$ et $R^2$ forment conjointement avec l'atome d'azote auquel ils sont liés un hétérocycle pentagonal à heptagonal saturé portant éventuellement un à trois substituants, identiques ou différents, alkyle ou hydroxyalkyle, chacun à chaîne droite ou ramifiée, avec 1 à 4 atomes de carbone, qui peut comporter 1 ou 2 autres atomes d'azote ou d'oxygène.

2. β-naphtylalkylamines suivant la revendication 1, dans la formule (I) desquels

Ar représente un reste β-naphtyle portant éventuellement un ou deux substituants, identiques ou différents, fluor, chlore, brome, hydroxy, méthyle, éthyle, n-propyle, isopropyle, n-butyle, isobutyle, sec.-butyle, tertiobutyle, méthoxy, éthoxy, n-propoxy ou isopropoxy,

$R^1$ et $R^2$, qui peuvent être identiques ou différents, représentent un groupe méthyle, éthyle, n-propyle, isopropyle, allyle, diméthylallyle, butényle, n-penténylе ou isopenténylе, ou bien

$R^1$ et $R^2$ forment conjointement avec l'atome d'azote auquel ils sont liés un hétérocycle portant éventuellement 1 à 3 substituants, identiques ou différents, méthyle, éthyle ou hydroxyméthyle, de formule

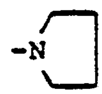 , 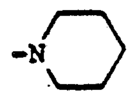 , 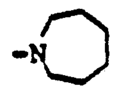 ou 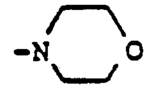

3. Procédé de production de β-naphtylalkylamines de formule (I)

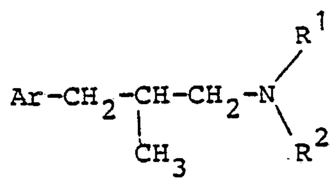

(I)

dans laquelle

Ar représente un reste β-naphtyle portant éventuellement un à trois substituants, identiques ou différents, halogéno, alkyle ou alkoxy ayant chacun 1 à 6 atomes de carbone,

$R^1$ et $R^2$, qui peuvent être identiques ou différents, représentent un groupe alkyle ou alcényle ayant chacun une chaîne droite ou ramifiée, avec jusqu'à 6 atomes de carbone, ou bien

$R^1$ et $R^2$ forment conjointement avec l'atome d'azote auquel ils sont liés un hétérocycle pentagonal à heptagonal saturé portant éventuellement un à trois substituants, identiques ou différents, alkyle ou hydroxyalkyle, chacun à chaîne droite ou ramifiée, avec 1 à 4 atomes de carbone, qui peut comporter 1 ou 2 autres atomes d'azote ou d'oxygène,

ainsi que de leurs sels d'addition d'acides compatibles avec les plantes, caractérisé en ce que :

(a) on fait réagir des composés β-naphtylalkyliques de formule (II)

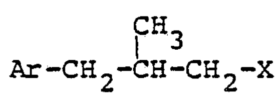

(II)

dans laquelle

Ar a la définition indiquée ci-dessus et

X est un groupe partant qui attire les électrons,

avec des amines de formule (III)

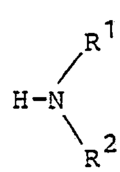

(III)

dans laquelle $R^1$ et $R^2$ ont la définition indiquée ci-dessus, le cas échéant en présence d'un diluant, et en la présence éventuelle d'un accepteur d'acide, ou bien

(b) on fait réagir des aldéhydes non saturés de formule (IV)

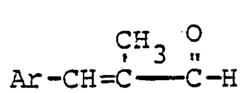

(IV)

dans laquelle Ar a la définition indiquée ci-dessus, avec des amines de formule (III)

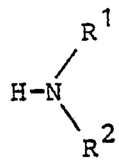

(III)

dans laquelle $R^1$ et $R^2$ ont la définition indiquée ci-dessus, en présence d'un agent réducteur et, le cas échéant, en présence d'un catalyseur de même que, éventuellement, en présence d'un diluant, et on ajoute ensuite éventuellement un acide.

4. Fongicides, caractérisés par une teneur en au moins une β-naphtylalkylamine de formule (I) suivant les revendications 1 et 3.

5. Utilisation de β-naphtylalkylamines de formule (I) suivant les revendications 1 et 3 pour combattre des champignons, excepté les utilisations non susceptibles de protection selon la loi.

6. Procédé pour combattre des champignons, caractérisé en ce qu'on fait agir des β-naphtylalkylamines de formule (I) suivant les revendications 1 et 3 sur des champignons et/ou sur leur milieu, excepté les utilisations non susceptibles de protection selon la loi.

7. Procédé de préparation de compositions fongicides, caractérisé en ce qu'on mélange des β-naphtylalkylamines de formules (I) suivant les revendications 1 et 3 avec des diluants et/ou des agents tensio-actifs.